# EUROPEAN PATENT APPLICATION

(11) **EP 2 325 718 A1**
(43) Date of publication of application: **25.05.2011**
(21) Application number: 09176969.5
(22) Date of filing: 24.11.2009
(51) Int. Cl.: G06F 1/16, A61B 5/00

(54) **Exchangeable Transparent Cover**

(71) Applicant: F. Hoffmann-La Roche AG, 4070 Basel (CH); Roche Diagnostics GmbH, 68305 Mannheim (DE)
(72) Inventor: Seiler, Thomas, 3427, Utzenstorf (CH)
(74) Representative: Schwabe - Sandmair - Marx

(57) **Abstract**

A transparent cover for a transparent area of an ambulatory medical device, wherein the transparent cover (1) is designed to be fixed to the transparent area in such a way as to allow the user to easily replace the transparent cover (1).

## Description

The invention relates to an exchangeable transparent cover for a transparent area of an ambulatory medical device, such as a display or a transparent window of a cartridge compartment of a medical pump, a glucose meter or the like. The exchangeable transparent cover is placed over the transparent area and can be affixed to the medical device by adhesive strength or by a positive fit or force fit.

Due to daily use, transparent areas of medical apparatus can easily become damaged, for example by scratching. Scratching can for example be caused by the strap arrangement which is used to carry the medical apparatus or by the tubes which touch the display during medication or touch other parts while the apparatus is being transported on a tray. Another typical cause of scratches and damage to the display is if the device in carried in trouser or jacket pockets, which is very common. Scratching decreases the quality of the data display, for example to the extent that it can hardly be read.

It is an object of the present application to maintain an unhindered view through a transparent area, despite damage such as scratching.

This object is solved by a transparent cover as disclosed in claim 1.

The invention relates to a transparent area, for example for a display of a medical device designed to be carried by the user or patient over extended time periods or even continuously in their everyday life on or near the body, for example on a belt or in a handbag, wherein the transparent cover is designed so as to be easily fixed to and removed from the top of the transparent area, in order to allow it to be easily and quickly exchanged, with or without the aid of a simple tool such as a knife. In some embodiments, the transparent cover has the same size or nearly the same size as the transparent area it is supplied for. If two or more separate transparent areas are present on a single device, a separate transparent cover can be provided for each transparent area, or alternatively, one transparent cover can be provided which covers some or all of the transparent areas of the device.

The transparent cover can comprise at least two layers, namely a first hard layer and a second soft layer, wherein the hard layer forms the outer surface of the transparent cover when it is fixed on a transparent area. The soft layer comprises a material which adheres to the transparent area, for example a display faceplate, or to the surface of the housing. In some embodiments, the soft layer is only supplied in peripheral regions of the first layer of the transparent cover; in other embodiments, it entirely covers one side of the first layer. The two layers can be attached to each other during production, in which case the soft layer can be covered with a removable protective layer, in order for example to prevent drying-out. It is also possible for the soft layer to not be applied to the hard layer until immediately prior to use, wherein the soft layer can be supplied in the form of a sheet which has the same size as the hard layer or which can be cut to this size. The soft layer can be provided in a tube or flask or any other suitable container as a pre-mixed paste or viscous fluid or as two or more components which have to be mixed before being applied.

Terminal strips can be provided in order to pre-define the position of the transparent cover on the transparent area or housing. The transparent cover can engage with the terminal strips, thus securing the transparent cover against falling off, even if the adhesive power of the soft layer deteriorates. The terminal strips additionally prevent the transparent cover from shifting laterally, at least perpendicular to the direction of the respective terminal strips.

In an alternative embodiment, the transparent cover comprises a frame which is connected to one or more of the outer ends of the hard layer. The frame can comprise a soft layer which will adhere to the transparent area, for example a display faceplate, or the surface of the housing. Additionally or alternatively, the frame can comprise at least one locking member which can engage with an aperture in the transparent area or with the housing which the transparent area forms a part of. If one or more locking members are provided, it is possible for an audible clicking noise to be produced when the locking member(s) engage(s) with the aperture, thus assuring the user that the transparent cover is in its predetermined position.

The locking member can comprise a predetermined breaking point. If the user wishes to remove the transparent cover from the display faceplate or the housing, they can use a knife or similar aid to break the predetermined breaking point. The parts of the locking member extending into the housing will either fall into a space inside the housing or will leave the housing through an opening, in order to clear the aperture so that a new transparent cover together with a frame comprising a locking member can be placed onto the transparent area or the housing, wherein the locking member of the new transparent cover engages with the same aperture.

As the transparent cover covers the whole transparent area, all the parts forming the transparent cover can be transparent, i.e. the hard layer, the soft layer and the frame can be made of a transparent material.

The hard layer can for example be made of or comprise glass, plastic, crystal or other suitable materials. In some embodiments, the material which the transparent cover is made of is harder than the material which the transparent area is made of. Thus, the transparent cover is more resistant to damage from scratching for example, resulting in a long service life before the transparent cover has to be exchanged. The hard layer can also have additional qualities such as a high resistance to aggressive fluids and heat.

The outer ends of the hard layer of the transparent cover can be bevelled so as not to exhibit any contact surfaces for forces which act on the projecting edges of the transparent cover and could thus displace the transparent cover and/or unintentionally break the predetermined breaking points of the locking members. In some embodiments, the angle of the bevel is between 30° and 60°. In some embodiments, the angle is substantially 45°.

The hard layer can be provided in any colour. It is thus possible to provide the user with a set of transparent covers of different colours, to allow the user to select a colour which best matches their style, particularly if the device comprising the transparent cover is to be carried in public.

The soft layer can be made of or comprise silicone, in order to fix the transparent cover to the transparent area or the housing. In some embodiments, the soft layer not only fixes the transparent cover but simultaneously seals the connection, to prevent dust or dirt invading the space between the transparent area and the transparent cover. The soft layer is thus applied, in a frame-like manner, to at least the outer edges of the hard layer or to a part of a frame which surrounds the transparent cover.

The transparent cover or at least the hard layer can be fluorescent or phosphorescent in order to help the user locate the transparent cover, and thus the device, in the dark. The transparent cover and/or the hard layer can for example be convex and thus exhibit a zooming function or serve as a magnifying glass, in order to render the display data more legible.

In a third embodiment of the invention, the transparent cover comprises a replaceable transparent film comprising an adhesive coating on one side. Like the hard layer described above, the transparent film can be any colour, can exhibit a greater resistance to scratching than the display faceplate, and can exhibit a zooming function. A frame can be provided on the device in order to position the film correctly on the transparent area or the housing. The film is either fixed to the frame, or the frame marks the region to which the film is to be fixed. The film can comprise two layers, namely a soft layer and a hard layer, wherein the latter is for example a transparent hard coating. Alternatively, the hard layer can be formed by thermally or chemically treating at least one side of the film, during or after production, in order to harden the surface in this region.

The invention also relates to a medical kit comprising an ambulatory medical device. The kit comprises, among other things, at least one transparent cover for a transparent area of the ambulatory medical device and/or any other device of the kit. In some embodiments, the kit comprises a set of transparent covers, wherein each transparent cover fits a certain transparent area of a device of the kit. The kit can also comprise sets of transparent covers of different colours in order to enable the user to select the colour which best matches their style and/or mood. As described above, the transparent covers are prepared for quick and easy exchange and can comprise a frame or can be formed so as to be inserted into a frame which forms part of the device. The kit can also comprise a simple dedicated tool to facilitate removing a worn transparent cover and replacing it with a new transparent cover.

The invention will now be illustrated on the basis of different embodiments. All the features described in connection with the figures and/or shown in the figures form part of the invention. The individual figures show:
- Figure 1: a first embodiment of the transparent cover; and
- Figure 2: a second embodiment of the transparent cover.

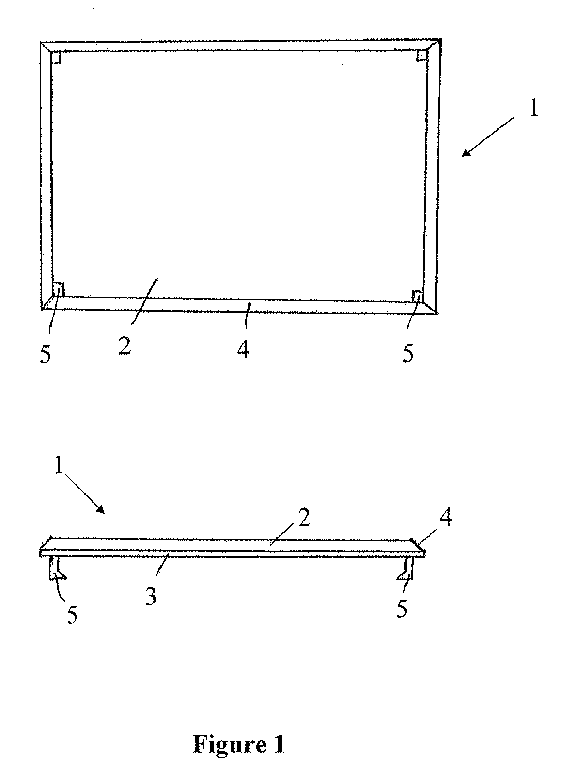

Figure 1 shows a first embodiment of the transparent cover, in the form of a display cover (1), in a top view and a lateral view. The display cover (1) comprises a hard layer (2) and a soft layer (3). The hard layer (2) forms the upper part of the display cover (1), while the soft layer (3) forms the lower part which directly contacts the housing or display faceplate (not shown) when it is in position. The hard layer (2) is made of a material which is harder than the material of the display faceplate which the display cover is to be fixed to. This material can be glass, for example a hardened glass, plastic or any other suitable material. The display cover can exhibit any colour and can be transparent in order to enable the user to read the data displayed on the display.

The hard layer exhibits a bevel (4) and locking members (5) which can engage with apertures (not shown) in a housing or display (not shown). The bevel (4) in the illustrated embodiment has an angle of about 60° but could alternatively exhibit any angle between 30° and 60°. In some embodiments, the angle of the bevel (4) is substantially 45°+ 5°.

The locking elements (5) can comprise a predetermined breaking point (not shown) which releases the display cover (1) if it is taken off the housing or the display faceplate. In some embodiments, the predetermined breaking point is formed directly on or just below the lower surface of the hard layer (2). If the display cover (1) has been removed, the lower part of the locking elements (5) can remain in the aperture, and the user can easily remove them from the housing, for example through an opening in the housing. Alternatively, the lower part of the locking means (5) can remain in the housing in a special container space which they automatically fall into when the display cover (1) is taken off.

The soft layer (3) can cover all of the lower surface of the hard layer (2) or can be applied to the lower surface only in the region of the bevel. The soft layer (3) is made of or comprises silicone or another suitable material which can reliably glue the display cover (1) to a base and simultaneously provide a reliable seal to prevent dust and dirt from invading the space between the display cover (1) and the base.

In the example shown, the soft layer (3) has been attached to the hard layer (2) during production or at least prior to delivery to the user. A hard layer (2) and a soft layer (3) can however also be delivered separately and not combined until immediately prior to gluing the display device (1) in place.

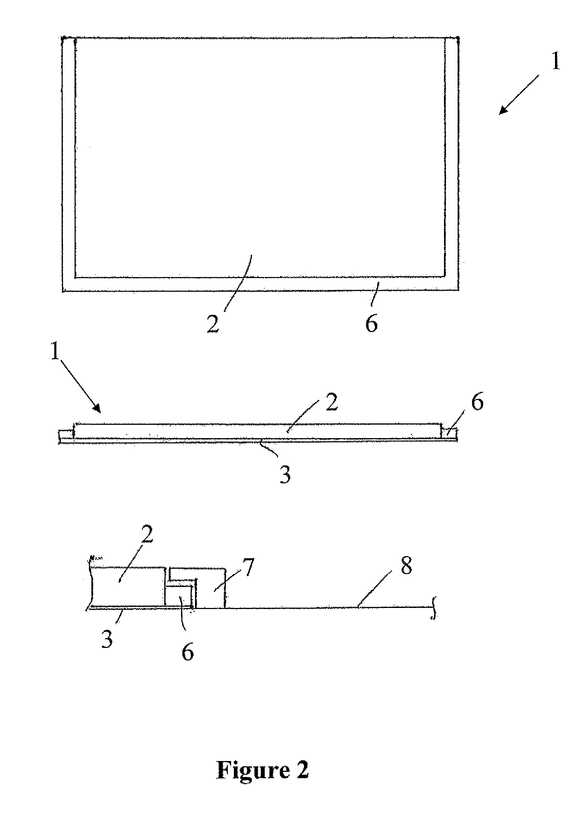

Figure 2 shows a second embodiment of the transparent cover in the form of a display cover (1). The display cover (1) of the second embodiment comprises a hard layer (2), a soft layer (3) and additionally a frame (6), which in the illustrated embodiment covers three sides of the hard layer (2). The frame (6) of the illustrated example has a different thickness than the hard layer (2). However, the frame (6) could equally well have the same thickness as the hard layer (2) or even a greater thickness than the hard layer (2). The frame (6) may be bevelled outwards, wherein the angle of the bevel can be freely chosen and can for example be about 45°.

The display cover (1) can be fixed to the base (8) using only the adhesive force of the soft layer (3). However, in order to additionally secure the display cover (1) on or above the display faceplate, terminal strips (7) can be provided which protrude from the surface (8) of the device and with which the frame (6) of the display cover (1) can engage in order to establish a secure connection between the display cover (1) and the display faceplate on the surface (8) of the device.

### List of reference signs

- 1: display cover
- 2: hard layer
- 3: soft layer
- 4: bevel
- 5: locking member
- 6: frame
- 7: terminal strip
- 8: base, surface

## Claims

1. A transparent cover for a transparent area of an ambulatory medical device, wherein the transparent cover (1) is designed to be fixed to the transparent area in such a way as to allow the user to easily replace the transparent cover (1).

2. The transparent cover according to claim 1, wherein the transparent cover (1) comprises a hard layer (2) and a soft layer (3) and wherein the soft layer (3) comprises a material which adheres to the transparent area.

3. The transparent cover according to claim 1 or claim 2, wherein terminal strips (7) are provided on a housing near the transparent area and wherein the transparent cover (1) gen-gages with the terminal strips (7).

4. The transparent cover according to any one of the preceding claims, wherein the transparent cover (1) comprises at least a hard layer (2) and a frame (6) connected to the outer ends of the hard layer (2), and wherein the frame (6) comprises a soft layer (3) which adheres to the transparent area and/or the frame (6) comprises at least one locking member (5) which engages with an aperture in a housing or in the transparent area.

5. The transparent cover according to claim 4, wherein the locking member (5) comprises a predetermined breaking point.

6. The transparent cover according to claim 4 or claim 5, wherein the frame (6) is transparent.

7. The transparent cover according to any one of claims 2 to 6, wherein the hard layer (2) of the transparent cover (1) is harder than an outer surface of the transparent area.

8. The transparent cover according to any one of the preceding claims, wherein the soft layer (3) is made of or comprises silicone, in order to fix the transparent cover (1) to the transparent area and to seal the transparent cover (1) to prevent dust or dirt from invading the space between the outer surface of the transparent area and the transparent cover (1).

9. The transparent cover according to any one of the preceding claims, wherein the outer ends of the hard layer (2) of the transparent cover (1) are bevelled.

10. The transparent cover according to any one of the preceding claims, wherein the transparent cover (1) serves as a magnifying glass or exhibits a zooming function.

11. The transparent cover according to claim 1, wherein the transparent cover (1) comprises a transparent film and an adhesive coating applied to one side of the film.

12. The transparent cover according to the preceding claim, wherein a frame or the like is provided on the device, in order to place the film in the correct position on the transparent area or a housing.

13. A kit, comprising an ambulatory medical device and at least one transparent cover (1) according to any one of claims 1 to 12, wherein the transparent cover (1) can be fixed to a transparent area of the ambulatory medical device.

14. The kit according to claim 13, wherein the kit comprises a set of transparent covers ( 1 ), wherein each transparent cover (1) fits a certain transparent area.

15. The kit according to claim 13 or 14, wherein the kit comprises a set of transparent covers of different colours for at least one of the transparent areas.

16. The kit according to any one of claims 13 to 15, wherein the kit further comprises a tool to facilitate removing a worn or damaged transparent cover (1).
